# EUROPÄISCHE PATENTSCHRIFT

(11) **EP 1 754 456 B1**
(45) Veröffentlichungstag und Bekanntmachung des Hinweises auf die Patenterteilung: **18.06.2008**
(21) Anmeldenummer: 05017771.6
(22) Anmeldetag: 16.08.2005
(51) Int. Cl.: A61F 2/38

(54) **Operationssystem**
Surgical system.
Système chirurgical.

(43) Veröffentlichungstag der Anmeldung: 21.02.2007
(73) Patentinhaber: Zimmer GmbH, 8404 Winterthur (CH)
(72) Erfinder: Ribic, Sonja, 8408 Winterthur (CH)
(74) Vertreter: Manitz, Finsterwald & Partner GbR

(56) Entgegenhaltungen:
- EP-A- 0 834 294
- DE-A1- 19 926 816
- US-A1- 2002 118 595
- US-A1- 2002 156 483

## Beschreibung

Die Erfindung betrifft ein Operationssystem mit wenigstens einem Implantat und Mitteln zum Befestigen des Implantats an einem Knochen, wobei die Befestigungsmittel zumindest ein festes, insbesondere aus ausgehärtetem Knochenzement bestehendes Distanzstück, das im implantierten Zustand zwischen dem Implantat und dem Knochen angeordnet ist, und aushärtbaren flüssigen Knochenzement umfassen, der in zwischen dem Distanzstück und dem Implantat und/oder zwischen dem Distanzstück und dem Knochen vorhandene Hohl- oder Zwischenräume einbringbar ist.

Derartige Operationssysteme kommen beispielsweise bei Knieoperationen zum Einsatz, bei denen künstliche Kniegelenke eingesetzt werden. Hierzu müssen entsprechend ausgebildete Implantate am Tibiaknochen und am Femurknochen befestigt werden. Bei den Implantaten handelt es sich insbesondere um so genannte Tibiaplattformen, die als Träger für Lagerschalen dienen, sowie um an die Lagerschalen angepasste Femurkondylenteile. Zur Vorbereitung der Implantatbefestigung werden die Knochen mit ebenen Resektionsflächen versehen.

Es ist bekannt, den Abstand zwischen den Implantaten und diesen Resektionsflächen mit Hilfe von so genannten "Spacern" einzustellen, die zwischen dem Knochen und dem Implantat platziert werden. Die Befestigung dieser Distanzstücke am Implantat und am Knochen erfolgt durch Einbringen von flüssigem Knochenzement in Hohlräume oder Zwischenräume, die durch entsprechende Ausgestaltung der Spacer zwischen diesem und dem Implantat bzw. der Resektionsfläche am Knochen vorhanden sind.

Flüssiger Knochenzement besitzt typische Aushärtzeiten von deutlich weniger als 30 Minuten und sorgt im ausgehärteten Zustand für eine formschlüssige Verbindung auch im Mikrostrukturbereich zwischen Spacer und Implantat bzw. Spacer und Knochen. Wichtige Eigenschaften von Knochenzement sind Körperverträglichkeit, gute Verarbeitbarkeit und die Fähigkeit, einerseits während der Einbringphase ausreichend lange fließfähig zu sein und andererseits nach dem Einbringen möglichst schnell auszuhärten.

Spacer aus Knochenzement sind in EP 834 294 A1 beschrieben. Im Gegensatz zu metallischen Spacern, die ohne Knochenzement am Implantat verankert sind, erzeugen mit Knochenzement am Implantat verankerte Spacer, wenn sie selbst aus Knochenzement bestehen, weniger Abrieb bei ihrer Nutzung. Voraussetzung dafür ist, dass die Hohl- und Zwischenräume zum Spacer so vollständig mit flüssigem Knochenzement ausgefüllt sind, dass durch das Anlösen mit flüssigem Knochenzement eine zugfeste Verbindung zum Spacer hergestellt wird.

Um den ehemals flüssigen Knochenzement im Röntgenbild erkennen zu können, ist es darüber hinaus bekannt, diesen mit einem geeigneten Kontrastmittel zu versehen und somit gewissermaßen "einzufärben". Die Konzentration oder der Anteil an Kontrastmittel wird dabei so gewählt, dass sich der ehemals flüssige Knochenzement im Röntgenbild gut von einem metallischen Implantat oder Spacer unterscheiden lässt.

Das Einfärben des flüssigen Knochenzements löst aber immer noch nicht das Problem, dass der Operateur im Röntgenbild nicht erkennen kann, ob der flüssige Knochenzement die vorhandenen Hohl- bzw. Zwischenräume zum Spacer vollständig ausfüllt, was für eine einwandfreie Befestigung des Implantats am Knochen erforderlich ist.

Die Zwischenkontrollen und periodischen Nachkontrollen im Anschluss an die Operation werden meist mit bildgebenden Verfahren, insbesondere mit Röntgenverfahren, durchgeführt. Es ist jedoch praktisch unmöglich, den Kontakt zwischen flüssigem Knochenzement und Distanzstück festzustellen, da ein Distanzstück aus Knochenzement von Luft und von seiner Umgebung nicht unterscheidbar ist.

Aufgabe der Erfindung ist es daher, ein Operationssystem der eingangs genannten Art derart weiterzubilden, dass der Operateur auf einfache und zuverlässige Weise kontrollieren kann, ob die Hohl- bzw. Zwischenräume in der gewünschten Weise mit Knochenzement gefüllt sind.

Die Lösung dieser Aufgabe erfolgt durch die Merkmale des Anspruchs 1.

Erfindungsgemäß sind das Distanzstück einerseits und der flüssige Knochenzement andererseits mit in Abhängigkeit von einem bei oder nach der Operation einzusetzenden bildgebenden Beobachtungs- oder Kontrollverfahren, insbesondere einem Röntgenverfahren, gewählten Kontrastmitteln derart versehen, dass das Distanzstück und der flüssige Knochenzement bei der Beobachtung oder Kontrolle ihrer Lage, insbesondere in einem Röntgenbild, von der Umgebung und voneinander unterscheidbar sind.

Die Erfindung basiert auf dem Gedanken, den flüssigen Knochenzement und das Distanzstück (Spacer) jeweils mit einem Kontrastmittel zu versehen, wobei dies aber in einer aufeinander abgestimmten Art und Weise erfolgt, um das Distanzstück vom flüssigen Knochenzement unterscheidbar zu machen.

Versuche mit bildgebenden Verfahren haben ergeben, dass es möglich ist, ohne Beeinträchtigung der mechanischen Eigenschaften des Knochenzements Kontrastmittel in einer für die Unterscheidung erforderlichen Art und Weise hinzuzufügen, d.h. genügend Kontrast zwischen Knochen, Spacer, Knochenzement und Implantat durch das "Einfärben" des Spacers mit Kontrastmittel zu erhalten.

Durch diese einfache und gleichzeitig äußerst wirkungsvolle Maßnahme wird erreicht, dass sich das Distanzstück einerseits und der flüssige Knochenzement andererseits insbesondere in einem Röntgenbild so weit voneinander unterscheiden, dass die betreffenden Komponenten durch ihre unterschiedliche "Einfärbung" oder "Schattierung" getrennt voneinander zu erkennen sind.

Es hat sich unerwartet herausgestellt, dass gemäß einer bevorzugten Ausführung der Erfindung das Distanzstück und der flüssige Knochenzement mit unterschiedlichen Konzentrationen bzw. Anteilen an insbesondere dem gleichen Kontrastmittel derart versehen werden können, dass im Röntgenbild ein ausreichender Kontrast zwischen Distanzstück und flüssigem Knochenzement besteht und gleichzeitig die Materialeigenschaften wie insbesondere deren Verarbeitbarkeit in keiner Weise beeinträchtigt werden.

In einem bevorzugten konkreten Ausführungsbeispiel, das sich für die Praxis als besonders geeignet herausgestellt hat, wird als Kontrastmittel sowohl für das Distanzstück als auch für den flüssigen Knochenzement Zirkonoxid verwendet, und zwar mit einem gewichtsbezogenen Anteil, der jeweils unter 10 % liegt (hierdurch ist eine einwandfreie Verarbeitbarkeit der Materialien gewährleistet), wobei der Konzentrationsunterschied zwischen Distanzstück und flüssigem Knochenzement wenigstens drei Gewichtsprozent beträgt. Das Distanzstück und der flüssige Knochenzement bestehen dabei aus dem gleichen Material, das vorzugsweise PMMA umfasst, wobei das Distanzstück etwa 2 bis 5 Gewichtsprozent Zirkonoxid und der flüssige Knochenzement etwa 5 bis 8 Gewichtsprozent Zirkonoxid enthält.

Allgemein ist erfindungsgemäß bevorzugt vorgesehen, dass sich das Distanzstück und der flüssige Knochenzement hinsichtlich ihres insbesondere gewichts- oder volumenbezogenen Anteils oder ihrer Konzentration an Kontrastmittel voneinander unterscheiden.

Es hat sich gezeigt, dass sowohl ein guter Kontrast als auch eine einwandfreie Materialverarbeitung gewährleistet werden können, wenn der Anteil bzw. die Konzentration an Kontrastmittel im Distanzstück geringer ist als im flüssigen Knochenzement. Damit lässt sich auch die Herstellung des Distanzstücks durch Spritzgießen problemlos durchführen.

Insbesondere aus Gründen der Verarbeitbarkeit wird ferner vorgeschlagen, dass der Anteil an Kontrastmittel im Distanzstück und im flüssigen Knochenzement jeweils einen Maximalwert von etwa 10 % nicht überschreitet.

Allgemein wurde festgestellt, dass gemäß einer weiteren bevorzugten Ausführung der Erfindung ein ausreichend guter Kontrast vorhanden ist, wenn die Differenz zwischen den beiden Kontrastmittelanteilen im Distanzstück und im flüssigen Knochenzement wenigstens etwa 3 % beträgt, wobei dies insbesondere bei Verwendung von Zirkonoxid als Kontrastmittel, Knochenzement auf PMMA-Basis, gewichtsbezogenen Anteilen und einem Röntgenverfahren gilt.

Obwohl nicht zwingend erforderlich, ist es bevorzugt, wenn das Distanzstück und der flüssige Knochenzement mit dem gleichen Kontrastmittel versehen sind.

Grundsätzlich sind beliebige Kontrastmittel verwendbar, wobei vorzugsweise als Kontrastmittel Zirkonoxid (ZrO₂) und/oder Bariumsulfat (BaSO₄) vorgesehen ist.

Obwohl prinzipiell beliebige Materialen in Frage kommen, sind das Distanzstück und/oder der flüssige Knochenzement vorzugsweise aus einem PMMA umfassenden Material hergestellt und umfassen insbesondere ein PMMA-Homopolymer, ein PMMA-Copolymer oder eine auf PMMA basierende Mischung, z. B. PMMA/PS (Polymethylmethacrylat/Polystyrol).

Bevorzugt sind das Distanzstück und der flüssige Knochenzement aus dem gleichen Material hergestellt.

Weitere bevorzugte Ausführungsbeispiele der Erfindung sind in den abhängigen Ansprüchen, der Beschreibung sowie der Zeichnung angegeben.

Die Erfindung wird im Folgenden beispielhaft unter Bezugnahme auf die Zeichnung beschrieben. Es zeigen
- Fig. 1 und 2: jeweils ein Beispiel für eine Anwendung des erfindungemäßen Operationssystems, nämlich an der Tibia (Fig. 1, Sagittalschnitt) und am Femur (Fig. 2, Sagittalschnitt),
- Fig. 3: einen Frontalschnitt zu Fig. 1, und
- Fig. 4: einen Schnitt analog zu Fig. 3 mit einem dickeren Distanzstück (Spacer).

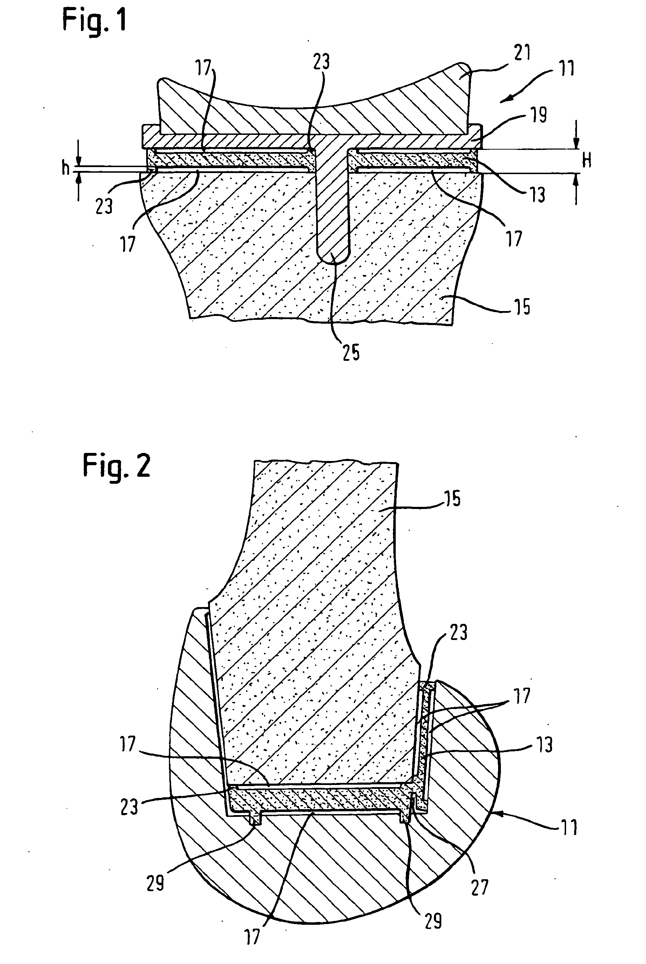

Fig. 1 zeigt das erfindungsgemäße Operationssystem an einem Tibiaknochen 15. Ein aus einer Lagerschale 21 und einer mit einer Stumpf 25 zur Verankerung im Knochen 15 versehenen Tibiaplattform 19 bestehendes Implantat 11 ist ein Bestandteil eines künstlichen Kniegelenkes. Ein weiterer Bestandteil dieses Gelenkes, nämlich ein mit der Lagerschale 21 zusammenwirkendes Kondylenprothesenteil an einem Femurknochen 15, ist in Fig. 2 dargestellt und bildet ebenfalls ein Implantat 11 im Sinne der Erfindung.

Zwischen den Implantaten 11 und den Knochen 15 ist jeweils ein im Folgenden auch als Spacer bezeichnetes, flächiges Distanzstück 13 angeordnet, das zur Lagekorrektur des jeweiligen Prothesenteils 11 am Knochen 15 dient und hierzu eine bestimmte Höhe bzw. Dicke H aufweist. Dem Operateur steht ein Satz von Spacern 13 unterschiedlicher Größe und Höhe H zur Verfügung. Wie Fig. 2 zeigt, können die Spacer 13 auch winklig ausgebildet sein.

In einem bevorzugten Ausführungsbeispiel bestehen die Spacer 13 jeweils aus ausgehärtetem Knochenzement auf PMMA-Basis.

Die Spacer 13 sind an ihrer Oberseite und Unterseite mit einer oder mehreren Vertiefungen versehen, wodurch Hohl- bzw. Zwischenräume 17 zwischen Spacer 13 und Knochen 15 bzw. zwischen Spacer 13 und Prothesenteil 11 vorhanden sind. An der Tibia (Fig. 1, 3 und 4) sind die Hohl- bzw. Zwischenräume 17 durch Vorsprünge 23 z.B. in Form einer sich über die gesamte jeweilige Fläche erstreckenden Rippenstruktur festgelegt.

Am Femur (Fig. 2) sind die Vorsprünge 23 jeweils als im Randbereich umlaufende Rippe ausgebildet, wodurch jeweils nur ein einziger Hohl- bzw. Zwischenraum 17 vorhanden ist.

Das winklige Distanzstück 13 gemäß Fig. 2 ist an einer seiner dem Implantat 11 zugewandten Seiten mit Zentrierstiften 29 versehen, die mit entsprechenden Aufnahmen im Prothesenteil 11 zusammenwirken, um auf diese Weise für eine Zentrierung des Distanzstücks 13 am Prothesenteil 11 zu sorgen.

Die Hohl- bzw. Zwischenräume 17 dienen zur Aufnahme von flüssigem Knochenzement, mit dem die Distanzstücke 13 am Knochen 15 und am Implantat 11 befestigt werden, um hierdurch die Implantate 11 fest mit dem Knochen 15 zu verbinden. Da die Distanzstücke 13 ebenfalls aus Knochenzement bestehen, entsteht nach dem Aushärten des in die Räume 17 eingebrachten flüssigen Knochenzements eine feste homogene Verbindung mit dem jeweiligen Spacer 13. Die Dicke der Zementschicht wird durch die Höhe h der Hohl- bzw. Zwischenräume bestimmt (Fig. 1).

Um zur Kontrolle der Operation im Röntgenbild die Spacer 13 und den flüssigen Knochenzement jeweils sowohl von der Umgebung als auch voneinander unterscheiden zu können, sind erfindungsgemäß sowohl der Spacer 13 als auch der flüssige Knochenzement "eingefärbt", d.h. mit einem Kontrastmittel versehen, wobei allerdings die Anteile an Kontrastmittel im Spacer 13 und im flüssigen Knochenzement unterschiedlich gewählt sind.

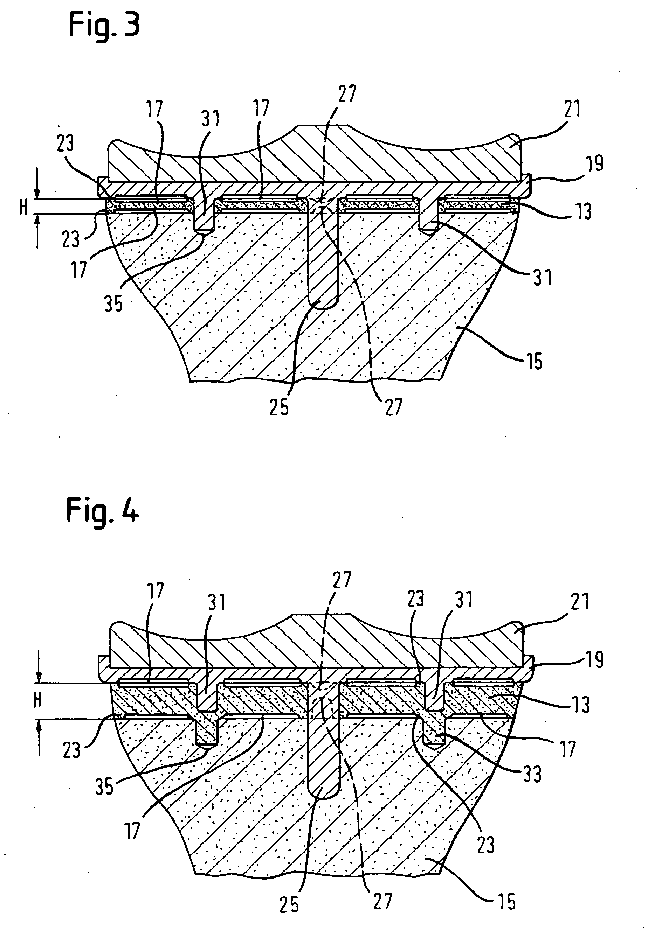

In Fig. 3 erkennt man, dass neben dem zentralen Stumpf 25 beidseitig Zentrierzapfen 31 von der Tibiaplattform 19 durch Aussparungen im Spacer 13 nach unten vorstehen, um einerseits den Spacer 13 zur Plattform 19 und andererseits Plattform 19 und Spacer 13 insgesamt bezüglich einer Drehung um den Stumpf 25 zum Knochen 15 auszurichten. Für die Zentrierzapfen 31 sind im Knochen 15 Bohrungen 35 vorgesehen.

Des Weiteren ist eine sagittal verlaufende Bruchkerbe 27 dargestellt, die kleinere Abmessungen aufweist als die Aussparung für den Stumpf 25 und die intraoperativ eine Halbierung zulässt, so dass auch nur eine Hälfte des Distanzstücks 13 durch Stumpf 25 und Zapfen 31 zentriert ist. Auf diese Weise können bei einem von lateral zu medial gestuften Resektionsschnitt linke und rechte Spacerhälften in einer entsprechend der Stufung unterschiedlichen Höhe H kombiniert werden, was die Anzahl der für die Herstellung notwendigen Spritzformen reduziert. Eine unterschiedliche Stufung kann sich ergeben, wenn man wirklich nur defektes Knochengewebe bei der Resektion berücksichtigt.

Es können links und rechts von den mittleren Bruchkerben 27 (Fig. 3) weitere, etwa parallel versetzt zu diesen verlaufende Bruchkerben vorgesehen werden, so dass die äußeren Teile des Distanzstücks auch bei monokondylären Anwendungen als Distanzstücke verwendet werden können.

In Fig. 4 ist ein Distanzstück 13 mit einer größeren Höhe H gezeigt. Entsprechend tiefer sind die Bruchkerben 27 ausgebildet. Dabei ist die Höhe H soviel größer als die vorstehenden Zapfen 31 der Plattform 19, dass am Spacer 13 eine Zapfenfortsetzung 33 in der Richtung der Zapfen 31 angebracht ist, wobei für die Zapfenfortsetzungen 33 im Knochen 15 Bohrungen 35 vorgesehen sind.

In Fig. 2 ist im Eckbereich des winkelförmigen Distanzstückes 13 ebenfalls eine Bruchkerbe 27 dargestellt, die es gestattet, intraoperativ - je nach Knochenzuschnitt - die Schenkel des winkelförmigen Distanzstückes 13 auch einzeln zu verwenden.

Auf die verschiedenen erfindungsgemäßen Möglichkeiten, das erfindungsgemäße Prinzip konkret in die Praxis umzusetzen, wurde bereits im Einleitungsteil eingegangen. Das folgende Ausführungsbeispiel hat sich in Versuchen als besonders geeignet herausgestellt:
Distanzstück (Spacer):
   - Material: Knochenzement auf PMMA-Basis
   - Kontrastmittel: 2 - 5 (bevorzugt 2) Gewichtsprozent Zirkonoxid
Flüssiger Knochenzement:
   - Material: wie Distanzstück
   - Kontrastmittel: 5 - 8 (bevorzugt 7) Gewichtsprozent Zirkonoxid

### Bezugszeichenliste

- 11: Implantat, Prothesenteil
- 13: Distanzstück, Spacer
- 15: Knochen
- 17: Hohl- bzw. Zwischenraum
- 19: Tibiaplattform
- 21: Lagerschale
- 23: Vorsprung
- 25: Stumpf
- 27: Bruchkerbe
- 29: Zentrierstift
- 31: Zentrierzapfen
- 33: Zapfenfortsetzung
- 35: Bohrung

- H: Höhe des Distanzelementes
- h: Höhe des Hohl- bzw. Zwischenraumes

## Patentansprüche

1. Operationssystem mit wenigstens einem Implantat (11) und Mitteln (13) zum Befestigen des Implantats (11) an einem Knochen (15),
wobei die Befestigungsmittel zumindest ein festes, insbesondere aus ausgehärtetem Knochenzement bestehendes Distanzstück (13), das im implantierten Zustand zwischen dem Implantat (11) und dem Knochen (15) angeordnet ist, sowie aushärtbaren flüssigen Knochenzement umfassen, der in zwischen dem Distanzstück (13) und dem Implantat (11) und/oder zwischen dem Distanzstück (13) und dem Knochen (15) vorhandene Hohl- oder Zwischenräume (17) einbringbar ist,
**dadurch gekennzeichnet, dass**
das Distanzstück (13) einerseits und der flüssige Knochenzement andererseits mit in Abhängigkeit von einem bei oder nach der Operation einzusetzenden bildgebenden Beobachtungs- oder Kontrollverfahren, insbesondere einem Röntgenverfahren, gewählten Kontrastmitteln derart versehen sind, dass das Distanzstück (13) und der flüssige Knochenzement bei der Beobachtung oder Kontrolle ihrer Lage, insbesondere in einem Röntgenbild, von der Umgebung und voneinander unterscheidbar sind.

2. Operationssystem nach Anspruch 1,
**dadurch gekennzeichnet ,**
**dass** sich das Distanzstück (13) und der flüssige Knochenzement hinsichtlich ihres insbesondere gewichts- oder volumenbezogenen Anteils oder ihrer Konzentration an Kontrastmittel voneinander unterscheiden.

3. Operationssystem nach Anspruch 1 oder 2,
**dadurch gekennzeichnet ,**
**dass** der Anteil oder die Konzentration an Kontrastmittel im Distanzstück (13) geringer ist als im flüssigen Knochenzement.

4. Operationssystem nach einem der vorhergehenden Ansprüche,
**dadurch gekennzeichnet ,**
**dass** der Anteil an Kontrastmittel im Distanzstück (13) und im flüssigen Knochenzement jeweils einen Maximalwert von etwa 10 % nicht überschreitet.

5. Operationssystem nach einem der Ansprüche 2 bis 4,
**dadurch gekennzeichnet ,**
**dass** die Differenz zwischen den beiden Kontrastmittelanteilen wenigstens etwa 3 % beträgt.

6. Operationssystem nach einem der vorhergehenden Ansprüche,
**dadurch gekennzeichnet ,**
**dass** das Distanzstück (13) und der flüssige Knochenzement mit dem gleichen Kontrastmittel versehen sind.

7. Operationssystem nach einem der vorhergehenden Ansprüche,
**dadurch gekennzeichnet ,**
**dass** als Kontrastmittel Zirkonoxid (ZrO₂) und/oder Bariumsulfat (BaSO₄) vorgesehen ist.

8. Operationssystem nach einem der vorhergehenden Ansprüche,
**dadurch gekennzeichnet ,**
**dass** das Distanzstück (13) etwa zwei bis fünf Gewichtsprozent, vorzugsweise etwa zwei Gewichtsprozent, Zirkonoxid oder Bariumsulfat und der flüssige Knochenzement etwa fünf bis acht Gewichtsprozent Zirkonoxid oder Bariumsulfat enthält.

9. Operationssystem nach einem der vorhergehenden Ansprüche,
**dadurch gekennzeichnet ,**
**dass** das Distanzstück (13) und/oder der flüssige Knochenzement aus einem PMMA umfassenden Material hergestellt ist und insbesondere ein PMMA-Homopolymer, ein PMMA-Copolymer oder eine auf PMMA basierende Mischung, z. B. PMMA/PS (Polymethylmethacrylat/Polystyrol), umfasst.

10. Operationssystem nach einem der vorhergehenden Ansprüche,
**dadurch gekennzeichnet ,**
**dass** das Distanzstück (13) und der flüssige Knochenzement - abgesehen von den Kontrastmitteln - aus dem gleichen Material hergestellt sind.

11. Operationssystem nach einem der vorhergehenden Ansprüche,
**dadurch gekennzeichnet ,**
**dass** das Distanzstück (13) mit einer oder mehreren Bruchkerben oder Sollbruchstellen (27) versehen ist, die intraoperativ eine Verkleinerung des Distanzstücks (13) auf eine jeweils erforderliche Dimension ermöglichen.

12. Operationssystem nach Anspruch 11,
**dadurch gekennzeichnet ,**
**dass** das Distanzstück (13) für eine Tibiaplattform an einem Kniegelenk ausgelegt und die Bruchkerben bzw. Sollbruchstellen (27) so auf dem Distanzstück (13) angelegt sind, dass das Distanzstück (13) sowohl für eine halbseitig gestufte als auch für eine monokondyläre Anwendung verwendbar ist.

13. Operationssystem nach einem der vorhergehenden Ansprüche,
**dadurch gekennzeichnet ,**
**dass** das Distanzstück (13) zum Knochen (15) hin einen oder mehrere Zentrierzapfen (33) aufweist, mit denen das Distanzstück (13) in im Knochen (15) vorgesehenen Bohrungen (35) ausrichtbar ist.

## Claims

1. An operation system having at least one implant (11) and means (13) for the fastening of the implant (11) to a bone (15),
wherein the fastening means include at least one fixed spacer (13), which in particular consists of hardened bone cement and is arranged between the implant (11) and the bone (15) in the implanted state, and hardenable liquid bone cement which can be introduced into hollow spaces or intermediate spaces (17) present between the spacer (13) and the implant (11) and/or between the spacer (13) and the bone (15),
**characterized in that**
the spacer (13), on the one hand, and the liquid bone cement, on the other hand, are provided with contrast agents chosen in dependence on an imaging observation process or monitoring process, in particular an X-ray process, to be used in or after the operation such that the spacer (13) and the liquid bone cement can be distinguished from the surroundings and from one another on the observation or monitoring of their position, in particular in an X-ray image.

2. An operation system in accordance with claim 1, **characterized in that** the spacer (13) and the liquid bone cement differ from one another with respect to their proportion, in particular related to weight or volume, or to their concentration of contrast agent.

3. An operation system in accordance with claim 1 or claim 2, **characterized in that** the proportion or the concentration of contrast agent in the spacer (13) is less than in the liquid bone cement.

4. An operation system in accordance with any one of the preceding claims, **characterized in that** the proportion of contrast agent in the spacer (13) and in the liquid bone cement in each case does not exceed a maximum value of approximately 10%.

5. An operation system in accordance with any one of the claims 2 to 4, **characterized in that** the difference between the two contrast agent proportions amounts to at least approximately 3%.

6. An operation system in accordance with any one of the preceding claims, **characterized in that** the spacer (13) and the liquid bone cement are provided with the same contrast agents.

7. An operation system in accordance with any one of the preceding claims, **characterized in that** zirconium oxide (ZrO₂) and/or barium sulfate (BaSO₄) is/are provided as the contrast agent.

8. An operation system in accordance with any one of the preceding claims, **characterized in that** the spacer (13) contains approximately two to five percent by weight, preferably approximately two percent by weight, zirconium oxide or barium sulfate and the liquid cement contains approximately five to eight percent by weight zirconium oxide or barium sulfate.

9. An operation system in accordance with any one of the preceding claims, **characterized in that** the spacer (13) and/or the liquid bone cement is/are manufactured from a material comprising PMMA and in particular includes/include a PMMA homopolymer, a PMMA copolymer or a mixture based on PMMA, e.g. PMMA/PS (polymethyl methacrylate polystyrene).

10. An operation system in accordance with any one of the preceding claims, **characterized in that** the spacer (13) and the liquid cement are manufactured from the same material - apart from the contrast agents.

11. An operation system in accordance with any one of the preceding claims, **characterized in that** the spacer (13) is provided with one or more break notches or desired break points (27) which permit a reduction of the spacer (13) to a respectively required dimension intraoperatively.

12. An operation system in accordance with claim 11, **characterized in that** the spacer (13) is designed for a tibial platform at a knee joint and the break notches or desired break points (27) are put into contact with the spacer (13) such that the spacer (13) can be used both for an application stepped on half one side and for a monocondylar application.

13. An operation system in accordance with any one of the preceding claims, **characterized in that** the spacer (13) has one or more centering pins (33) toward the bone (15) with which the spacer (13) can be aligned in bores (35) provided in the bone (15).

## Revendications

1. Système opératoire comprenant au moins un implant (11) et des moyens (13) pour fixer l'implant (11) sur un os (15), dans lequel les moyens de fixation comprennent au moins une pièce d'écartement solide (13) constituée en particulier de ciment osseux durci, qui est agencée dans l'état implanté entre l'implant (11) et l'os (15), ainsi que du ciment osseux liquide durcissable, susceptible d'être introduit dans les cavités ou intervalles (17) présent(e)s entre la pièce d'écartement (13) et l'implant (11) et/ou entre la pièce d'écartement (13) et l'os (15),
**caractérisé en ce que**
la pièce d'écartement (13) d'une part et le ciment osseux liquide d'autre part sont dotés d'agents de contraste sélectionnés en fonction d'un procédé d'observation ou de contrôle, en particulier un procédé par rayons X, formant des images et à utiliser pendant ou après l'opération, de telle manière que la pièce d'écartement (13) et le ciment osseux liquide peuvent être distingués par rapport à l'environnement et l'un par rapport à l'autre lors de l'observation ou du contrôle de leur position, en particulier dans une image aux rayons X.

2. Système opératoire selon la revendication 1,
**caractérisé en ce que** la pièce d'écartement (13) et le ciment osseux liquide se distinguent l'un par rapport à l'autre en ce qui concerne leurs proportions, en particulier par référence au poids ou au volume, ou leur concentration en agent de contraste.

3. Système opératoire selon la revendication 1 ou 2,
**caractérisé en ce que** la proportion ou la concentration d'agent de contraste dans la pièce d'écartement (13) est plus faible que dans le ciment osseux liquide.

4. Système opératoire selon l'une des revendications précédentes,
**caractérisé en ce que** la proportion d'agent de contraste dans la pièce d'écartement (13) et dans le ciment osseux liquide ne dépasse respectivement pas une valeur maximum d'environ 10 %.

5. Système opératoire selon l'une des revendications 2 à 4,
**caractérisé en ce que** la différence entre les deux proportions d'agent de contraste s'élève au moins à environ 3 %.

6. Système opératoire selon l'une des revendications précédentes,
**caractérisé en ce que** la pièce d'écartement (13) et le ciment osseux liquide sont dotés du même agent de contraste.

7. Système opératoire selon l'une des revendications précédentes,
**caractérisé en ce que** l'on prévoit à titre d'agent de contraste de l'oxyde de zirconium (ZrO₂) et/ou du sulfate de baryum (BaSO₄).

8. Système opératoire selon l'une des revendications précédentes,
**caractérisé en ce que** la pièce d'écartement (13) contient environ 2 à 5 % en poids, de préférence environ 2 % en poids, d'oxyde de zirconium ou de sulfate de baryum, et le ciment osseux liquide contient environ 5 à 8 % en poids d'oxyde de zirconium ou de sulfate de baryum.

9. Système opératoire selon l'une des revendications précédentes,
**caractérisé en ce que** la pièce d'écartement (13) et/ou le ciment osseux liquide est réalisé à partir d'un matériau comprenant du PMMA, et en particulier un homopolymère PMMA, un copolymère PMMA, ou un mélange à base de PMMA, par exemple PMMA/PS (polyméthylméthacrylate/polystyrène).

10. Système opératoire selon l'une des revendications précédentes,
**caractérisé en ce que** la pièce d'écartement (13) et le ciment osseux liquide sont réalisés à partir du même matériau, à l'exception des agents de contraste.

11. Système opératoire selon l'une des revendications précédentes,
**caractérisé en ce que** la pièce d'écartement (13) est pourvue d'une ou plusieurs entailles de rupture ou d'un ou plusieurs emplacements de rupture de consigne (27) qui permettent en cours d'opération de rapetisser la pièce d'écartement (13) à une dimension respectivement nécessaire.

12. Système opératoire selon la revendication 11,
**caractérisé en ce que** la pièce d'écartement (13) est conçue pour une plate-forme tibiale dans une articulation du genou, et les entailles de rupture ou les emplacements de rupture de consigne (27) sont placé(e)s sur la pièce d'écartement (13) de telle façon que la pièce d'écartement (13) est utilisable aussi bien pour une application en gradins sur un demi-côté que pour une application à monocondyle.

13. Système opératoire selon l'une des revendications précédentes,
**caractérisé en ce que** la pièce d'écartement (13) comprend un ou plusieurs tenons de centrage (33) en direction de l'os (15), au moyen desquels la pièce d'écartement (13) peut être orientée dans des perçages (35) prévus dans l'os (15).
